# EUROPÄISCHE PATENTANMELDUNG

(11) **EP 1 709 937 A1**
(43) Veröffentlichungstag der Anmeldung: **11.10.2006**
(21) Anmeldenummer: 06006101.7
(22) Anmeldetag: 24.03.2006
(51) Int. Cl.: A61C 8/00, A61B 17/86

(54) **Schraubenförmiges Dentalimplantat**

(30) Priorität: 05.04.2005 DE 202005005421 U
(71) Anmelder: Dinkelacker, Wolfgang, Dr. med. dent., 71065 Sindelfingen (DE)
(72) Erfinder: Dinkelacker, Wolfgang, Dr. med. dent., 71065 Sindelfingen (DE)
(74) Vertreter: Kindermann, Manfred

(57) **Zusammenfassung**

Ein schraubenförmiges Dentalimplantat weist ein mehrgängiges Gewinde am Umfang des Implantatkörpers (11) auf, dessen Gewindegänge (30, 31) eine variable Tiefe haben, die sich über den Umfang des Implantatkörpers kontinuierlich verändert. Die Tiefe der Gewindegänge verläuft über den Umfang des Implantatkörpers wellenförmig wenigstens einmal von einer minimalen Tiefe zu einer maximalen Tiefe und von dort zurück zu einer minimalen Tiefe. Das Profil der Gewindegänge ist in seiner Bemessung an die Größe der sich an der Implantatoberfläche anlagernden Osteonen des Kieferknochens angepasst. Der Implantatkörper weist einen profilierten Implantatkopf (20), einen zylindrischen Gewindeabschnitt (12) sowie einen kegligen Gewindeabschnitt (14) aufweist, der selbstschneidend ausgebildet ist.

## Beschreibung

### Bereich der Erfindung

Die Erfindung bezieht sich auf ein operativ in den menschlichen Knochen einschraubbares Dentalimplantat mit einem zylindrischen Implantatkörper, der an seinem Umfang ein feingängiges Gewinde aufweist.

### Stand der Technik

Es sind Knochenimplantate mit einem schraubenförmigen Implantatkörper bekannt, die in ein entsprechend bemessenes Loch im menschlichen Knochen eingeschraubt werden. Das Loch wird entsprechend dem Kerndurchmesser des Schraubengewindes bemessen, so dass mit dem Einschrauben des Implantats dessen sichere Verankerung im Knochen erreicht wird. Das Einschrauben wird erleichtert, wenn das Schraubengewinde selbstschneidend ausgebildet ist. Hierzu werden in das Schraubengewinde Schneidkannten eingearbeitet sind, so dass es nach Art eines Gewindebohrers wirkt. Derartige Implantate können vorzugsweise als Kieferimplantate ausgebildet sein (z.B. EP 1 234 550 A1).

Des weiteren ist ein schraubenförmiges Knochenimplantat bekannt, dessen Schraubengewinde rillenförmige Vertiefungen aufweist, die im Gewindegang angeordnet sind (WO 2004/098442 A1). Die Größe der rillenförmigen Vertiefungen ist an die Größe der Osteone des Knochengewebes angepasst, die sich an die rillenförmigen Vertiefungen anlagern. Die rillenförmigen Vertiefungen verlaufen vorzugsweise in einer Richtung, die im wesentlichen der Richtung des Gewindeganges entspricht. Sie bilden ein dem Schraubengewinde überlagertes Mikrogewinde, das die Kontaktfläche zwischen Implantat und Knochengewebe vergrößert und die Anlagerung der Osteonen an der Oberfläche des in den Knochen eingeschraubten Implantats unterstützt.

Es ist bei schraubenförmigen Dentalimplantaten auch bekannt, zur besseren Verankerung des Implantats im Kieferknochen über den Umfang des Implantatkörpers verteilte axial verlaufende Kanäle anzubringen, durch die die Gewindeflanken unterbrochen und in Segmente unterteilt werden (US 5,427,527) oder in einem Gewindeabschnitt des Implantatkörpers an den Gewindeflanken eine Mehrzahl von Abflachungen vorzusehen, die über den Umfang des Gewindeabschnitts verteilt sind und Raum für die Anlagerung von Knochenmaterial bieten (DE 20 2004 018 855).

Es ist ferner bekannt, die Oberfläche eines Dentalimplantats mit einer Vielzahl in Richtung seiner Längsachse oder in einem spitzen Winkel zu dieser verlaufenden rillenförmigen Vertiefungen auszustatten, die radial zur Oberfläche des Implantatkörpers geneigt sind und über ihre Länge eine unterschiedliche Tiefe aufweisen (DE 201 13 183.8). Die rillenförmigen Vertiefungen sind in ihrem Grundriss keilförmig oder rautenförmig ausgebildet und begünstigen die Anlagerung der Osteonen des Knochengewebes an die Oberflache des Implantats.

### Zusammenfassung der Erfindung

Durch die vorliegende Erfindung wird die Verankerung eines schraubenförmigen Dentalimplantats im Kieferknochen weiter verbessert.

Gemäß der Erfindung, wie sie in den Ansprüchen definiert ist, weist das Implantatkörper ein Gewinde an seinem Umfang auf, das wenigstens einen Gewindegang besitzt, dessen Tiefe sich über den Umfang des Implantatkörpers verändert.

Eine bevorzugte Ausbildung der Erfindung besteht darin, dass die Tiefe des Gewindegangs über den Umfang des Implantatkörpers wellenförmig wenigstens einmal von einer minimalen Tiefe zu einer maximalen Tiefe und von dort zurück zu einer minimalen Tiefe variiert. Die Tiefe des Gewindegangs kann über 180 Grad des Umfangs des Implantatkörpers einmal von der minimalen Tiefe zur maximalen Tiefe und von dort zurück zur minimalen Tiefe variieren.

Nach einer Ausführungsform der Erfindung sind im Verlauf des Gewindegangs über den Umfang des Implantatkörpers Bereiche größter Tiefe den Bereichen kleinster Tiefe benachbart.

Das Gewinde ist vorzugsweise ein Trapezgewinde, das mehrere Gänge aufweist, wobei im Verlauf benachbarter Gewindegänge über den Umfang des Implantatkörpers Bereiche größter Tiefe und Breite den Bereichen kleinster Tiefe und Breite benachbart sind. Die Tiefe und Breite der Gewindegänge variiert über 180 Grad des Umfangs des Implantatkörpers einmal von der minimalen Tiefe zur maximalen Tiefe und von dort zurück zur minimalen Tiefe.

Das Profil der Gewindegänge ist gemäß weiterer Ausbildung der Erfindung in seiner Bemessung an den Größenbereich der sich an der Implantatoberfläche anlagernden Osteonen angepasst.

Dentalimplantate mit Schraubgewinde haben den Vorteil, dass sie die Ausbreitung von Gewebeinfektionen in Richtung des Implantatkopfes verhindern oder zumindest erschweren. Diese Wirkung wird erfindungsgemäß kombiniert mit der durch die Gewindegänge mit variabler Tiefe und Breite hervorgerufene Wirkung, die Anlagerung der Knochenzellen zu erleichtern und zu fördern. Diese Wirkung ergibt sich aus dem inhärenten Verhalten der Kochenzellen, insbesondere der Osteonen, sich an die erfindungsgemäß ausgebildeten Gewindegänge variabler Tiefe besser anzulagern, als dies bei konventionellen Gewinden der Fall ist. Durch die Erfindung wird eine schnelle und nachhaltige Osteointegration der Implantate erreicht.

### Beschreibung der Zeichnungen

Nachfolgend ist eine bevorzugte Ausführungsformen der Erfindung anhand von Zeichnungen dargestellt. Es zeigen:
Figur 1 die Seitenansicht einer Ausführungsform eines schraubenförmigen Dentalimplantats gemäß der Erfindung;
Figur 2 eine Schnittdarstellung entlang der Linie A-A in Figur 1;
Figur 3 eine Darstellung des Implantatkopfes in Richtung F in Figur 1;
Figur 4 eine vergrößerte Schnittdarstellung entlang der Linie C-C in Figur 1;
Figur 5 eine vergrößerte Schnittdarstellung entlang der Linie B-B in Figur 1;
Figur 6 eine vergrößerte Detailansicht des Gewindeprofils bei G in Figur 2;
Figur 7 eine vergrößerte Schnittdarstellung entlang der Linie D-D in Figur 1;
Figur 8 eine vergrößerte Schnittdarstellung entlang der Linie E-E in Figur 1; und
Figur 9 eine vergrößerte Detailansicht des Gewindeprofils bei H in Figur 2.

### Detaillierte Beschreibung des in den Zeichnungen dargestellten Ausführungsbeispiels der Erfindung

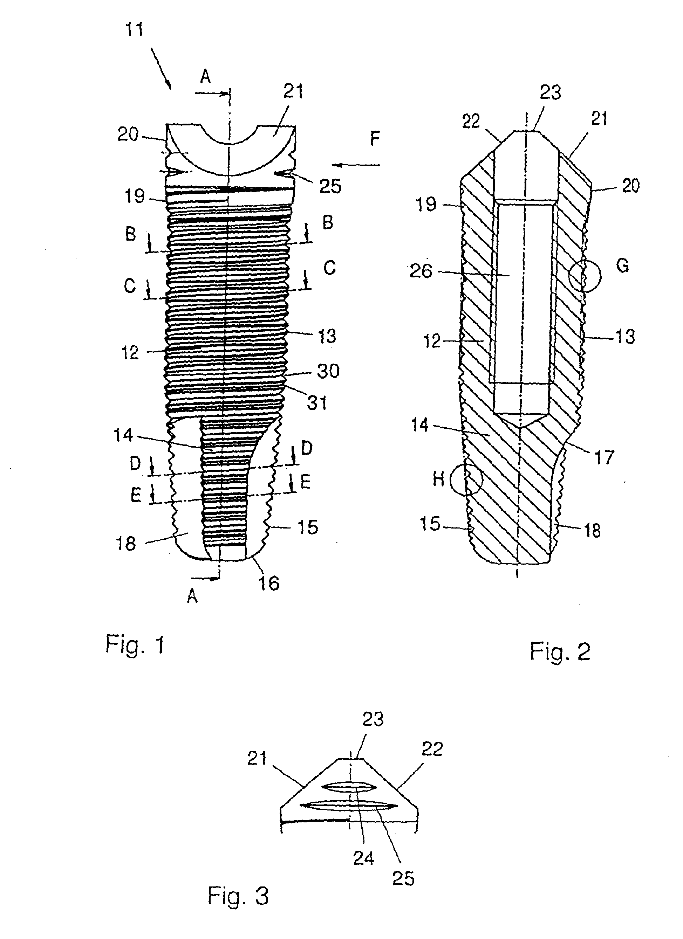

Das in Figur 1 dargestellte Dentalimplantat umfaßt einen Implantatkörper 11, der als selbstschneidende Schraube ausgebildet ist. Der Implantatkörper 11 weist einen zylindrischen mittleren Gewindeabschnitt 12 auf, der ein feingängiges Außengewinde 13 aufweist. An den zylindrischen Gewindeabschnitts 12 schließt sich in apikaler Richtung ein kegliger Gewindeabschnitt 14 an, über den sich der Implantatkörper 11 verjüngt und der an seinem Umfang ebenfalls ein Schraubengewinde 15 aufweist. An seinem apikalen Ende 16 ist der Implantatkörper abgerundet. Der keglige Abschnitt 14 ist in für sich bekannter Weise als selbstschneidende Schraube ausgebildet, wozu der Implantatkörper 11 im Bereich des Gewindeabschnitts 14 drei in axialer Richtung verlaufende und um 120 Grad versetzte Ausnehmungen 17 aufweist, von denen jede durch eine in Einschraubrichtung wirkende Schneidkante 18 begrenzt ist.

Am anderen Ende des zylindrischen Gewindeabschnitts 13 vergrößert sich der Durchmesser des Implantatkörpers 11 durch einen kurzen kegelförmigen Übergangsabschnitt 19 zu einem zylindrischen Implantatkopf 20, der den crestalen Abschluß des Implantatkörpers 11 bildet. Der Implantatkopf 20 weist ein Profil in Form von zwei sich gegenüberliegenden Abschrägungen 21, 22 und einer Dachfläche 23 auf, durch die der Implantatkopf an die Anatomie des Kieferkammes angepasst wird. Im Bereich zwischen den Abschrägungen 21, 22 sind Rillen 24, 25 angeordnet, die sich über den Umfang des Implantatkopfes 20 erstrecken und vor den Abschrägungen 21, 22 in den Umfang des Implantatkopfes 20 auslaufen. Wie aus Figur 3 ersichtlich, sind die Rillen 24, 25 aufgrund des dachförmigen Profils des Implantatkopfes 20 unterschiedlich lang.

Die Oberfläche des Implantatkörpers 11 ist biokompatibel ausgebildet. Sie kann durch eine Oberflächenbehandlung eine Mikrorauhigkeit erhalten, die die Integration des Implantats im Knochengewebe des Kieferknochens und im Gingivagewebe fördert. Die Oberfläche des Implantatkörpers 11 kann in den mit Knochengewebe oder Weichgewebe in Kontakt kommenden Bereichen mit einem biokompatiblen Material beschichtet sein oder durch Säureätzen oder Sandstrahlen behandelt werden. Die behandelten Bereiche können zusätzlich durch eine Sputterschicht bedeckt werden, die vorzugsweise aus Titan besteht. Die Oberflächenbehandlung kann auf den Implantatkopf 20 ausgedehnt werden, wobei die sich gegenüberliegenden Abschrägungen 21, 22 und die Dachfläche 23 unbehandelt bleiben.

Der Implantatkörper 11 besitzt eine zentrale Gewindebohrung 26, in die eine Schraube eingreift, durch die ein nichtgezeigter Implantataufsatz mit dem Implantatkörper 11 verbunden wird. Der Implantataufsatz dient als Träger für eine Zahnkrone oder einen anderen Zahnersatz. Die Schraube und der Implantataufsatz sind in den Zeichnungen nicht dargestellt. Der Implantatkörper 11 besteht vorzugsweise aus Titan oder einer Titanlegierung, aus Keramik oder aus einem anderen mit dem Kieferknochengewebe verträglichen Material ausreichender Härte.

Das Gewinde 13 am Umfang des zylindrischen Abschnitts 12 besitzt mehrere Gewindegänge, die in den kurzen kegelförmigen Übergangsabschnitt 19 auslaufen. Die Gewindegänge weisen über ihre Länge eine variable Tiefe auf. Über den Umfang des Implantatkörpers 11 verläuft die Tiefe der Gewindegänge wellenförmig. Die Tiefe der Gewindegänge verändert sich über den Umfang des Implantatkörpers wenigstens zweimal kontinuierlich von einer minimalen Tiefe zu einer maximalen Tiefe und von dort zurück zu einer minimalen Tiefe. Dementsprechend bildet der Tiefenverlauf über des Implantatkörperumfangs jeweils über 360 Grad des Implantatkörperumfangs zwei Wellentäler und zwei Wellenberge. Hierbei sind die einander benachbarten Gewindegänge um 90 Grad gegeneinander versetzt, so dass dem Wellental eines Gewindegangs ein Wellenberg des benachbarten Gewindegangs benachbart ist.

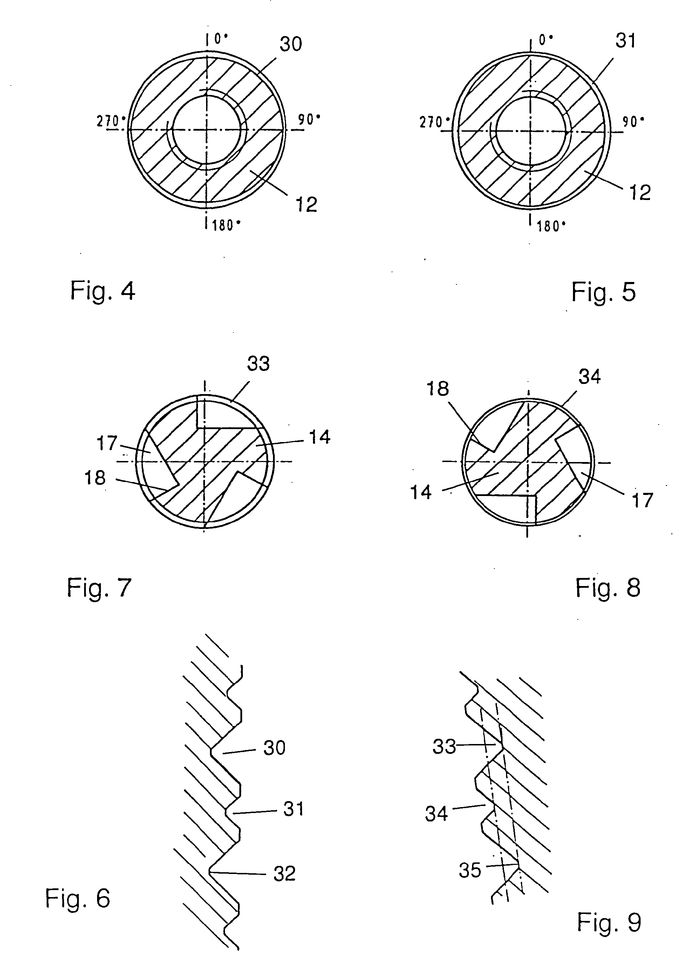

Ein Ausführungsbeispiel für diesen Gewindeverlauf ist in den Figuren veranschaulicht. Der zylindrische Abschnitt 12 des Implantatkörper 11 weist ein zweigängiges Gewinde mit den Gewindegängen 30, 31 auf. Die Schnittdarstellung von Figur 4 zeigt den Tiefenverlauf des ersten Gewindeganges 30 über den Umfang des Implantatkörpers 11. Der erste Gewindegang 30 weist bei 0 Grad seine größte Tiefe auf. Die Tiefe verringert sich bis zu ihren niedrigsten Wert bei 90 Grad und steigt danach wieder an bis zum Maximalwert bei 180 Grad, von wo sie sich erneut bis zu ihren niedrigsten Wert bei 270 Grad verringert. Die Figur 5 zeigt den Tiefenverlauf des benachbarten zweiten Gewindeganges 31 über den Umfang des Implantatkörpers 11. Der zweite Gewindegang weist bei 0 Grad seine geringste Tiefe auf. Die Tiefe vergrößert sich bis zu ihrem höchsten Wert bei 90 Grad. Sie verringert sich danach wieder bis zum Minimalwert bei 180 Grad und erreicht erneut ihren Maximalwert bei 270 Grad. Im Verlauf beider Gewindegänge 30, 31 über den Umfang des Implantatkörpers 11 sind somit Bereiche größter Tiefe den Bereichen kleinster Tiefe benachbart.

Im dargestellten Ausführungsbeispiel besitzt das Gewinde 13 in beiden Gewindegängen 30, 31 einen einheitlichen Flankenwinkel. Mit Änderung der Tiefe der Gewindegänge 30, 31 ändert sich auch deren Breite. In Figur 6 ist das Profil der Gewindegänge 30, 31 dargestellt. Die Figur 6 zeigt die Gewindegänge 30, 31 in Bezug auf die Figuren 4 und 5 bei 0 Grad, wo der Gewindegang 30 seine größte Tiefe und Breite aufweist, während der benachbarte Gewindegang 31 dort seine kleinste Tiefe und Breite aufweist.

Das Gewinde des Implantatkörpers 11 ist ein Feingewinde, bei dem die Bemessung der Gewindegänge an die Größe der sich am Implantatkörper 11 anlagernden Knochenzellen angepasst sind. Der sich aus dem sich erfindungsgemäß verändernden Tiefen der Gewindegänge ergebende radial geneigte Verlauf der Gewindegänge begünstigt die Anlagerung der Knochenzellen und führt zu einer schnellen und dauerhafte Verankerung des Implantats im Kieferknochen.

Die Tiefe der Gewindegänge liegt in einem Bereich von 10-200 Mikrometern, d.h., dass ihre maximale Tiefe bis zu 200 Mikrometer und ihre minimale Tiefe wenigstens 10 Mikrometer beträgt. In dem in den Figuren 7-9 dargestellten Ausführungsbeispiel wird für die Gewindegänge 30, 31 ein Tiefenbereich von 70-150 Mikrometern bevorzugt, für den die maximale Tiefe 150 Mikrometer und die minimale Tiefe 70 Mikrometer beträgt. Der Flankenwinkel der Gewindegänge 30, 31 liegt im Bereich von 80 bis 90 Grad. Für das trapezförmiges Profil des dargestellten Ausführungsbeispiels wird ein Flankenwinkel von 88 Grad bevorzugt. Das Profil der Gewindegänge 30, 31 besitzt eine schmale Grundfläche 32, die parallel zur Implantatachse ausgerichtet ist und die eine Ausdehnung von vorzugsweise 30 Mikrometern besitzt. Durch die im Ausführungsbeispiel vorgesehene Dimensionierung der Gewindegänge 30, 31 werden diese an den Größenbereich der fadenförmigen Osteonen angepasst, deren Durchmesser im Bereich zwischen 20 und 300 Mikrometern liegt.

Das Gewinde 15 des kegelförmigen Abschnitts weist die gleiche Steigung wie das Gewinde 13 des zylindrischen Abschnitts auf und ist ebenfalls ein zweigängiges Gewinde mit den Gewindegängen 33, 34. Das Gewinde 15 besitzt jedoch eine andere Struktur als das Gewinde 13. Jeder der beiden Gewindegänge 33, 34 hat über seine Länge eine einheitliche Tiefe und benachbarte Gewindegänge sind unterschiedlich tief. Ebenso wie das Gewinde 13 ist auch das Gewinde 15 an die Größe der sich am Implantatkörper 11 anlagernden Knochenzellen angepasst. Die Tiefe der Gewindegänge liegt in einem Bereich von 50-200 Mikrometern. Im dargestellten Ausführungsbeispiel hat der erste der beiden Gewindegänge 33, 34 eine Tiefe von 154 Mikrometern und der zweite dieser Gewindegänge eine Tiefe von 75 Mikrometern. Die Gewindegänge 33, 34 besitzen ebenfalls ein trapezförmiges Profil, dessen Flankenwinkel im Bereich von 80 bis 90 Grad liegt. Beim dargestellten Ausführungsbeispiel wird ein Flankenwinkel von 88 Grad bevorzugt. Des weiteren weisen die Gewindegänge 33, 34 eine Grundfläche 35 von vorzugsweise 30 Mikrometern auf. Damit liegen die Gewindegänge 33, 34 wie auch die Gewindegänge 31, 32 im Größenbereich der Osteonen.

Während die Erfindung anhand eines bevorzugten Ausführungsbeispiels beschrieben wurde, können Abwandlungen und andere Ausführungsformen realisiert werden, ohne daß dadurch der durch die Ansprüche definierte Bereich verlassen wird. So können z.B. die Gewinde 13 und 15 anstatt zwei Gänge eine größere Anzahl von Gewindegängen aufweisen oder auch als eingängiges Gewinde ausgebildet sein. Bei Verwendung von mehr als zwei Gewindegängen sind im kegelförmigen Abschnitt 14 tiefe Gewindegänge im Wechsel mit flacheren Gewindegängen angeordnet. Des weiteren können die Gewinde 13 und 14 ein anderes Profil aufweisen und z.B. als Spitzgewinde oder als Rundgewinde ausgebildet sein. Auch die Abmessungen der Gewindegänge können von den oben angegebenen Abmessungen abweichen.

## Patentansprüche

1. Dentalimplantat mit einem Implantatkörper (11), der über wenigsten einen Teil seines Umfangs zylindrisch ausgebildet ist und ein feingängiges Gewinde (13) mit wenigstens einem Gewindegang aufweist, **dadurch gekennzeichnet, dass** sich die Tiefe des Gewindegangs (30) über den Umfang des Implantatkörpers (11) verändert.

2. Implantat nach Anspruch 1, **dadurch gekennzeichnet, dass** die Tiefe des Gewindegangs (30) über den Umfang des Implantatkörpers (11) wellenförmig wenigstens einmal von einer minimalen Tiefe zu einer maximalen Tiefe und von dort zurück zu einer minimalen Tiefe variiert.

3. Implantat nach Anspruch 2, **dadurch gekennzeichnet, dass** die Tiefe des Gewindegangs (30) über 180 Grad des Umfangs des Implantatkörpers (11) einmal von der minimalen Tiefe zur maximalen Tiefe und von dort zurück zur minimalen Tiefe variiert.

4. Implantat nach Anspruch 1 und 2, **dadurch gekennzeichnet, dass** sich im Verlauf des Gewindegangs (30) über den Umfang des Implantatkörpers Bereiche größter Tiefe den Bereichen kleinster Tiefe benachbart sind.

5. Implantat nach einem der Ansprüche 1-4, **dadurch gekennzeichnet, dass** das Gewinde (13) ein Trapezgewinde ist.

6. Implantat nach einem der Ansprüche 1-5, **dadurch gekennzeichnet, dass** das Gewinde (13) mehrere Gänge (30, 31) aufweist.

7. Implantat nach Anspruch 6, **dadurch gekennzeichnet, dass** im Verlauf benachbarter Gewindegänge (30, 31) über den Umfang des Implantatkörpers (11) Bereiche größter Tiefe und Breite den Bereichen kleinster Tiefe und Breite einander benachbart sind.

8. Implantat nach Anspruch 6, **dadurch gekennzeichnet, dass** die Tiefe und Breite der Gewindegänge (30, 31) über 180 Grad des Umfangs des Implantatkörpers einmal von der minimalen Tiefe und Breite zur maximalen Tiefe und Breite und von dort zurück zur minimalen Tiefe und Breite variiert.

9. Implantat nach Anspruch 6, **dadurch gekennzeichnet, dass** Gewinde (13) zwei Gewindegänge (30, 31) aufweist.

10. Implantat nach einem der Ansprüche 1-9, **dadurch gekennzeichnet, dass** das Profil der Gewindegänge (30, 31) in seiner Bemessung an den Größenbereich der sich an der Implantatoberfläche anlagernden Osteonen angepasst wird.

11. Implantat nach einem der Ansprüche 1-10, **dadurch gekennzeichnet, dass** die Gangtiefe der Gewindegänge (30, 31) im Bereich von 10 bis 200 Mikrometern liegt.

12. Implantat nach Anspruch 11, **dadurch gekennzeichnet, dass** die Gangtiefe der Gewindegänge (30, 31) im Bereich von 50 bis 150 Mikrometern liegt.

13. Implantat nach Anspruch 11, **dadurch gekennzeichnet, dass** der Flankenwinkel der Gewindegänge (30, 31) im Bereich von 80 bis 90 Grad liegt.

14. Implantat nach Anspruch 1, **dadurch gekennzeichnet, dass** dass der Implantatkörper (11) einen zylindrischen Gewindeabschnitt (12) aufweist, der an seinem Umfang mit einem feingängigen Gewinde (13) ausgestattet ist, dass sich an den zylindrischen Gewindeabschnitt in apikaler Richtung ein kegelförmiger Gewindeabschnitt (14) anschließt, der selbstschneidend ausgebildet ist, und dass sich am anderen Ende des zylindrischen Abschnitt ein kegelförmiger Abschnitt (19) anschließt, in den die Gewindegänge (30, 31) des Gewindes (13) auslaufen und der den Übergang zu einem Implantatkopf (20) bildet.

15. Implantat nach Anspruch 14, **dadurch gekennzeichnet, dass** der apikale kegelförmige Abschnitt (14) an seinem Umfang ein Gewinde (15) mit im wesentlicher gleicher Steigung und Gangzahl wie das Gewinde (13) des zylindrischen Abschnitts (12) aufweist.

16. Implantat nach Anspruch 15, **dadurch gekennzeichnet, dass** und dass die Gewindegänge (33, 34) des Gewindes (15) über ihre Länge eine einheitliche Tiefe aufweisen und dass benachbarte Gewindegänge unterschiedlich tief sind.

17. Implantat nach Anspruch 14, **dadurch gekennzeichnet, dass** der Implantatkörper (11) einen profilierten Implantatkopf (20) mit beidseitigen Abschrägungen (21, 22) aufweist.

18. Implantat nach einem der Ansprüche 14-17, **dadurch gekennzeichnet, dass** der Implantatkopf (20) an seinem Umfang als Zylinder ausgebildet ist, der sich an den kegelförmigen Abschnitt (19) anschließt und der im Bereich zwischen den beidseitigen Abschrägungen (21, 22) Rillen (24, 25) aufweist, die quer zur Implantatachse verlaufen.

19. Implantat nach einem der Ansprüche 1-18, **dadurch gekennzeichnet, dass** der Implantatkörper (11) eine biokompatible Oberfläche aufweist.
